# EUROPÄISCHE PATENTANMELDUNG

(11) **EP 1 035 407 A2**
(43) Veröffentlichungstag der Anmeldung: **13.09.2000**
(21) Anmeldenummer: 00104326.4
(22) Anmeldetag: 02.03.2000
(51) Int. Cl.: G01N 1/20

(54) **Probeentnahmevorrichtung in einer Milchsammelanlage**

(30) Priorität: 05.03.1999 DE 19909655
(71) Anmelder: JANSKY GMBH, D-48282 Emsdetten (DE)
(72) Erfinder: Jansky, Manfred, 48282 Emsdetten (DE)
(74) Vertreter: Cohausz & Florack

(57) **Zusammenfassung**

Die Erfindung betrifft eine Probeentnahmevorrichtung in einer Milchsammelanlage, bei der in einem ersten Behälter bereitgestellte Milch über eine Förderleitung (2) in einen zweiten Behälter (3) gefördert wird. Dabei wird ein mengenproportionaler Teilstrom abgezweigt und in einen Probeentnahme- und Mischbehälter (13, 14) geleitet, aus dem einzelne Probegefäße befüllt werden. Zur Verbesserung des Repräsentativitätsfaktors der Milchprobe erfolgt vor jeder Abzweigung eine Vorspülung der Probeentnahme- und Mischbehälter (13, 14).

## Beschreibung

Die Erfindung betrifft eine Probeentnahmevorrichtung in einer Milchsammelanlage mit einer von einem ersten Behälter zu einem zweiten Behälter führenden Förderleitung und mindestens einer davon mit einem bestimmten Teilungsverhältnis abzweigenden und zu einem Probeentnahme- und Mischbehälter führenden Abzweigleitung, und einer von dem Probeentnahme- und Mischbehälter zu der weiterführenden Förderleitung führenden Entleerungsleitung oder direkt zu dem zweiten Behälter.

Es ist üblich, mit einer insbesondere auf einem Wagen aufgebauten Milchsammelanlage Milch unterschiedlicher Volumina und unterschiedlicher Qualität bei Lieferanten in Empfang zu nehmen. Milchsammelanlagen, die mit einem Volumenmesser und einer Probeentnahmevorrichtung ausgerüstet sind (EP 0 217 155 B1), sind bekannt. Die Probeentnahmevorrichtung ermöglicht es, mehr oder weniger exakt eine repräsentative Probe für die beim jeweiligen Lieferanten anzunehmende Milch zu erstellen. Dies geschieht in der Weise, daß während des gesamten Annahmevorganges ein Teilstrom aus dem Hauptstrom abgezweigt und in einen kleinen Probeentnahme- und Mischbehälter gefüllt wird, aus dem am Ende des Annahmevorganges ein kleineres Volumen als Milchprobe in ein Probegefäß abgefüllt wird. Nach Erstellen der Probe wird die übrige im Probeentnahme- und Mischbehälter verbleibende Milch der bereits in einem Sammeltank angenommenen Milch zugegeben. Diese Art der Probeentnahme ist sowohl bei Milchsammelanlagen, die mit Vakuum die Milch in den Sammeltank der Milchsammelanlage fördern, als auch bei Milchsammelanlagen, die mit einer Förderpumpe die Milch in den Sammeltank fördern, üblich.

Bei einer bekannten Probeentnahmevorrichtung der eingangs genannten Art (DE-GM 6600291) liegt der Einlaß der Abzweigleitung für das mengenproportional abzuzweigende Volumen für die Probe im Milchstrom der Förderleitung, die wie im übrigen Bereich einen runden oder quadratischen Querschnitt hat. Die Abzweigleitung liegt quer zur Förderleitung und erstreckt sich über deren gesamten Durchmesser bzw. Höhe. Als Einlaß dient ein Längsschlitz in der Abzweigleitung, dessen Länge dem Durchmesser bzw. der Höhe der Förderleitung entspricht. Dieser Anordnung liegt der Gedanke zugrunde, daß es für eine repräsentative Probe nötig ist, über den gesamten Durchmesser der Förderleitung Milch mengenprortional zu entnehmen. In diesem Sinne wird es als besonders vorteilhaft angesehen, wenn die Milch vor dem Eintritt in den Einlaß der Abzweigleitung in Turbulenz versetzt worden ist. Nach Kenntnis der fachkundigen Anmelderin hat sich diese Art der Entnahme einer repräsentativen Probe in der Praxis aber nicht durchgesetzt, vielmehr hat man in der jüngsten Vergangenheit versucht, den Repräsentativitätsfaktor der Probe auf andere Art und Weise zu verbessern.

Um der Forderung nach einem möglichst hohen Repräsentativitätsfaktor zu genügen, hat man in der Vergangenheit verschiedene Anstrengungen unternommen. So hat man vor Befüllung der Probegefäße mit der mengenproportional abgezweigten und im Probeentnahme- und Mischbehälter gesammelten Milch eine Durchspülung der zu den Probegefäßen führenden Leitungen einschließlich eines eingebauten Ventils mit einem Teil dieser Milch vorgenommen, um an den Wandungen dieser Teile der Probeentnahmevorrichtung haftende Restmengen aus der jeweils vorherigen Annahme zu entfernen. Diese Art der Durchspülung zur Vermeidung sogenannter Verschleppungsfehler ist aber völlig unzureichend, weil an den Wandungen der übrigen Leitungen und vor allem des Probeentnahme- und Mischbehälters haftende Milch aus der vorherigen Annahme nicht erfaßt wird (EP-A 098966). Auch hat man versucht, für unterschiedliche Volumina der anzunehmenden Milch mit unterschiedlichen Proportionalitätsfaktoren abzuzweigen, um zu gewährleisten, daß über die gesamte Phase der Annahme Milch abgezweigt wird (EP 0 217 155 B1). Dagegen hat man keine Anstrengungen hinsichtlich eines anderen Aufbaus im Bereich des Einlasses der Abzweigung unternommen. Offensichtlich war die Fachwelt der Auffassung, daß es in dieser Hinsicht nichts zu verbessern gibt.

Der Erfindung liegt die Aufgabe zugrunde, eine bezüglich des Repräsentativitätsfaktors verbesserte Probeentnahmevorrichtung bei einfachem konstruktiven Aufbau zu schaffen.

Diese Aufgabe wird bei einer Probeentnahmevorrichtung der eingangs genannten Art dadurch gelöst, daß eine absperrbare Spülleitung, die von der Förderleitung ausgeht und zu dem Probeentnahme- und Mischbehälter führt, einen wesentlich größeren Strömungsquerschnitt und/oder kürzeren Strömungsweg als die Abzweigleitung hat.

Mit der erfindungsgemäßen Probeentnahmeeinrichtung werden Proben mit einem Repräsentativitätsfaktor entnommen, wie er bisher nicht erzielt werden konnte. Bei der erfindungsgemäßen Probeentnahmevorrichtung sorgt der vergrößerte Querschnitt und/oder der kürzere Weg dafür, daß diejenigen an der Probeentnahme beteiligten Leitungen und Behälter, an deren Wandungen haftende Milch vor allem für Verschleppungsfehler verantwortlich ist, von der anzunehmenden Milch durchspült werden, bevor der mengenproportional abgezweigte Anteil in den Probeentnahme- und Mischbehälter gelangt. Da die Milch für die Spülung und für die mengenproportionale Probenentnahme parallel entnommen wird, geht über keine Phase der Annahme Milch für die Probeentnahme verloren.

Der schon wegen der Vermeidung von Verschleppungsfehler verbesserte Repräsentativitätsfaktor kann weiter durch die Merkmale der Unteransprüche verbessert werden.

Vorzugsweise ist der in einem Abschnitt mit rechteckigem Querschnitt der Förderleitung liegende Einlaß der Abzweigleitung als gekrümmtes Rohrstück ausgebildet, dessen offenes Ende der Strömungsrichtung der Milch in der Förderleitung entgegengerichtet ist.

Die mit dieser Ausgestaltung verbundene höhere Repräsentativität ist auf die gleichzeitige Anwendung von rechteckigem Querschnitt und gekrümmtem Rohrstück als Einlaß zurückzuführen, das mit ihrem offenen Ende der Strömungsrichtung entgegengerichtet ist. Bei einem rechteckigen Querschnitt ergibt sich im Vergleich zu einem runden Querschnitt bei gleicher Höhe bzw. Durchmesser eine geringere Strömungsgeschwindigkeit und eine gleichmäßigere Strömung des Förderstroms.

Anders als beim beschriebenen Stand der Technik liegt der Eingang der Abzweigleitung also nicht in einem turbulenten Strömungsbereich, sondern in einem strömungsberuhigten Bereich. Da es schon beim Ansaugen der Milch eine ausreichende Durchmischung gibt, erübrigt sich an der Stelle der Probenentnahme eine erneute Durchmischung, wie sie im Stand der Technik für vorteilhaft gehalten wurde. Turbulente Strömungen an der Entnahmestelle stören nämlich eine mengenproportionale Abzweigung, die maßgebend den Repräsentationsfaktor beeinflußt.

Wie an sich bekannt, ermöglicht die erfindungsgemäße Probeentnahmevorrichtung auch die Einstellung auf unterschiedliche Proportionalitätsfaktoren. Im Fall von zwei verschiedenen Proportionalitätsfaktoren ist nach einer Ausgestaltung vorgesehen, daß eine zweite Abzweigleitung, die zu einem zweiten Probeentnahme- und Mischbehälter führt, mit einem auf ein anderes Teilungsverhältnis eingestellten, als gekrümmtes Rohrstück ausgebildeten Einlaß gleichgerichtet neben dem einen Einlaß angeordnet ist.

Um nach Beendigung der Annahme der Milch Probegefäße zu füllen, sieht eine Ausgestaltung der Erfindung vor, daß von jedem Probeentnahme- und Mischbehälter eine Fülleitung mit integrierter Dosierpumpe ausgeht und zu einer Abfüllvorrichtung für Probegefäße führt.

Um bei unterschiedlichen Prinzipien (Annahme mit Unterdruck oder Annahme mit Pumpe) den Probeentnahme- und Mischbehälter möglichst unbeeinflußt von der Anlage selbst repräsentativ zu füllen, ist nach einer weiteren Ausgestaltung der Erfindung eine Druckausgleichsleitung für den Arbeitsdruck der Milchsammelanlage angeschlossen.

Im folgenden wird die Erfindung anhand einer ein Ausführungsbeispiel darstellenden Zeichnung näher erläutert. Im einzelnen zeigen:
- Figur 1: einen Teil einer mit Unterdruck arbeitenden Milchsammelanlage in schematischer Darstellung
**und**
- Figur 2: einen Abschnitt einer Förderleitung für die anzunehmende Milch mit Abzweigleitungen für eine Milchprobe und einer Spülleitung in isometrischer und vergrößerter Darstellung.

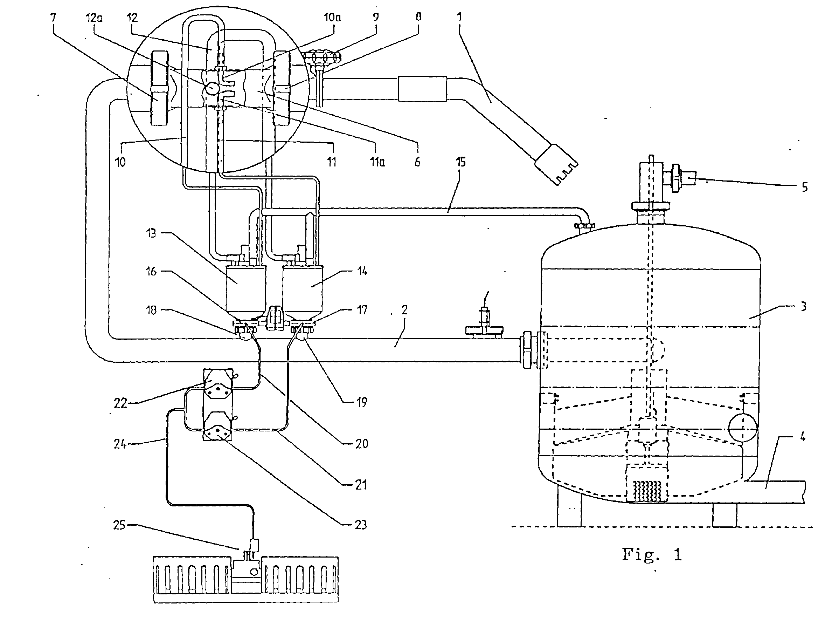

Bei dem in Figur 1 dargestellten Teil der Milchsammelanlage wird die von einem Lieferanten in einem nicht dargestellten ersten Behälter zur Annahme bereitgestellte Milch über einen in diesen ersten Behälter einzutauchenden Saugrüssel 1 und eine Förderleitung 2 in einen zweiten Behälter 3 gefördert. Vom Boden dieses zweiten Behälters 3 gelangt die Milch über eine weitere Leitung 4 zu einem nicht dargestellten Sammeltank. Dies geschieht mittels einer in der Leitung 4 angeordneten, nicht dargestellten Pumpe.

Die Förderung der Milch aus dem ersten Behälter in den zweiten Behälter 3 erfolgt mittels Unterdruck. Dazu ist der obere Teil des Behälters 3 über eine Leitung 5 an einer nicht dargestellten Unterdruckquelle angeschlossen. Der unter Unterdruck stehende zweite Behälter 3 wirkt als Luftabscheider für die über die Förderleitung 2 unter Einschluß von Luft und Schaum in den zweiten Behälter 3 geförderte Milch. Über die Leitung 4 kann dann die Milch frei von Lufteinschlüssen in den Sammeltank gefördert und dabei vollvolumetrisch gemessen werden.

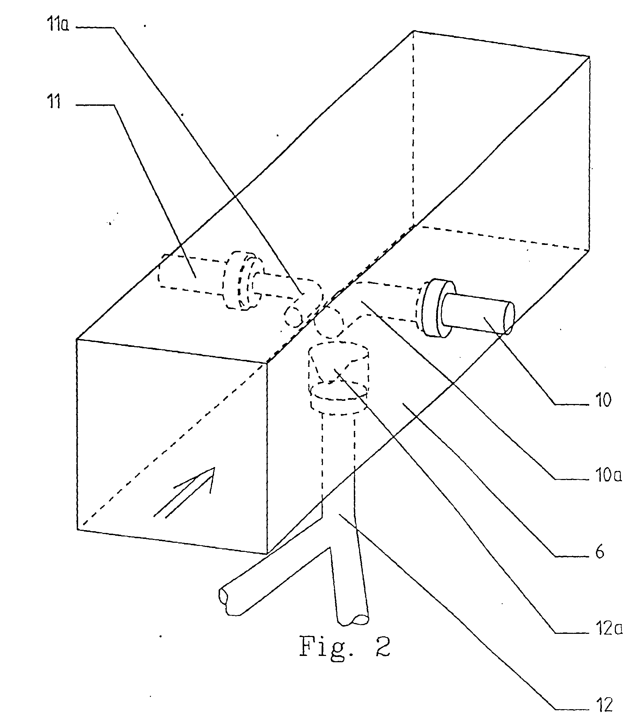

Der Förderleitung 2 ist eine Probeentnahmevorrichtung zugeordnet. Diese Probeentnahmevorrichtung weist, wie in Fig. 2 auch in vergrößertem Maßstab dargestellt ist, einen Abschnitt 6 mit im wesentlichen quadratischem Querschnitt auf, der über Verbindungsflansche 7,8 in der Förderleitung 2 in Strömungsrichtung der Milch hinter einem Absperrventil 9 eingebaut ist. In gegenüberliegenden Seitenwänden dieses Abschnittes 6 sind zwei Abzweigleitungen 10, 11 mit dem Milchstrom entgegengerichteten Einlässen 10a, 11a montiert. Dazu sind die Einlässe 10a, 11a als gekrümmte Rohrstücke ausgebildet, deren offene Enden der Strömungsrichtung der Milch in der Förderleitung 2 entgegengerichtet sind. Die Einlässe 10a, 11a haben einen unterschiedlichen Öffnungsquerschnitt, um mit unterschiedlichen Proportionalitätsfaktoren Milch abzuzweigen.Von der Förderleitung 2 zweigt ferner eine mit einem nicht dargestellten Ventil absperrbare Spülleitung 12 ab, die einen um ein Vielfaches größeren Strömungsquerschnitt als die Abzweigleitungen 10, 11 hat. Sowohl die Abzweigleitungen 10, 11 als auch die Spülleitung 12 münden in Probeentnahme- und Mischbehälter 13, 14, die über eine Druckausgleichsleitung 15 mit dem zweiten Behälter 3 verbunden sind. Über Ventile 16, 17 zu öffnende Entleerungsleitungen 18, 19 münden in die Förderleitung 2. Alternativ könnten sie auch direkt in den Behälter 3 münden.

Von den Probeentnahme- und Mischbehältern 13, 14 gehen Fülleitungen 20, 21 aus, in denen insbesondere als Schlauchpumpen ausgebildete Dosierpumpen 22, 23 angeordnet sind. Von hier führt eine gemeinsame weitere Fülleitung 24 zu einer Abfülleinrichtung 25 für einzelne Probegefäße.

Die Arbeitsweise der Probeentnahmevorrichtung ist folgende:
Am Anfang der Annahme von Milch wird über die Abzweigleitungen 10, 11 und die Spülleitung 12 parallel aus der Förderleitung 2 Milch abgezweigt und den Probeentnahme- und Mischbehältern 13, 14 zugefördert. Wegen des größeren Querschnittes der Spülleitung 12 gelangt allerdings zuerst die über die Spülleitung 12 abgezweigte Milch in die Probeentnahme- und Mischbehältern 13, 14 und wird aus diesen wieder abgelassen und in die Förderleitung 2 eingespeist, bevor die mit bestimmten Proportionalitätsfaktoren abgezweigte Milch die so vorgespülten Probeentnahme- und Mischbehälter 13, 14 erreicht. Für die schnelle, der Zuführung der Probenmilch vorgeschaltete Befüllung der Probeentnahme- und Mischbehälter 13, 14 ist der große Strömungsquerschnitt maßgebend. Ein kurzer Strömungsweg und eine örtlich sehr frühe Entnahme aus der Förderleitung 2 wirken sich ebenfalls günstig aus, um den Spülvorgang der Probeentnahme- und Mischbehälter 13, 14 rechtzeitig vor deren Befüllung mit Probenmilch abzuschließen. Mittels des nicht dargestellten Ventils wird die Spülleitung 12 gesperrt, wenn für die Spülung genügend Milch abgezweigt worden ist. So wird erreicht, daß nach Durchspülung und Entleerung der beteiligten Leitungen und vor allem der Probeentnahme- und Mischbehälter 13, 14 die Probeentnahme- und Mischbehälter 13, 14 ohne Verschleppungsfehler nur mit den eingestellten Proportionalitätsfaktoren entsprechenden Milchvolumina befüllt werden.

Es versteht sich, daß der Einsatz der Probeentnahme- und Mischbehältern 13, 14 nicht auf das beschriebene Ausführungsbeispiel einer Milchsammelanlage, die mit Unterdruck die Milch fördert, beschränkt ist, sondern grundsätzlich auch für andere Milchsammelanlagen gilt, insbesondere für solche, bei denen die Milch mittels einer Pumpe in der Förderleitung gefördert wird. Auch müssen nicht beide Maßnahmen, die Abzweigung der Milch mittels der Strömungrichtung der Milch entgegengerichteten Einlässen (10a, 11a) aus der Förderleitung (2) mit rechteckigem Querschnitt und die Vorspülung, gemeinsam verwirklicht sein. Jede einzelne Maßnahme verbessert schon den Repräsentativitätsfaktor.

## Patentansprüche

1. Probenentnahmevorrichtung in einer Milchsammelanlage mit einer von einem ersten Behälter zu einem zweiten Behälter (3) führenden Förderleitung (2) und mindestens mindestens einer davon mit einem bestimmten Teilungsverhältnis abzweigenden und zu einem Probeentnahme- und Mischbehälter (12,13) führenden Abzweigleitung (10, 11), und einer von dem Probeentnahme- und Mischbehälter (12,13 zu der weiterführenden Förderleitung (2) führenden Entleerungsleitung (18, 19) oder direkt in den zweiten Behälter (3),
**dadurch gekennzeichnet, daß** eine absperrbare Spülleitung (12), die von der Förderleitung (2) ausgeht und zu dem Probeentnahme- und Mischbehälter (13, 14) führt, einen wesentlich größeren Strömungsquerschnitt und/oder kürzeren Strömungsweg als die Abzweigleitung (10, 11) hat.

2. Probeentnahmevorrichtung nach Anspruch 1,
**dadurch gekennzeichnet, daß** der in einem Abschnitt (6) mit rechteckigem Querschnitt der Förderleitung (2) liegende Einlaß (10a) der Abzweigleitung (10) als gekrümmtes Rohrstück ausgebildet ist, dessen offenes Ende der Strömungsrichtung der Milch in der Förderleitung (2) entgegengerichtet ist.

3. Probeentnahmevorrichtung nach Anspruch 1 oder 2,
**dadurch gekennzeichnet, daß** eine zweite Abzweigleitung (11), die zu einem zweiten Probenahme- und Mischbehälter (14) führt, mit einem auf ein anderes Teilungsverhältnis eingestellten, als gekrümmtes Rohrstück ausgebildeten Einlaß (11a) gleichgerichtet neben dem einen Einlaß (10a) angeordnet ist.

4. Probeentnahmevorrichtung nach einem der Ansprüche 1 bis 3,
**dadurch gekennzeichnet, daß** der rechteckige Querschnitt des Abschnittes (6) der Förderleitung (2) quadratisch ist.

5. Probeentnahmevorrichtung nach einem der Ansprüche 1 bis 4,
**dadurch gekennzeichnet, daß** von jedem Probeentnahme- und Mischbehälter (13, 14) eine Fülleitung (20, 21) mit integrierter Dosierpumpe (22, 23) ausgeht und zu einer Abfüllvorrichtung (25) für Probegefäße führt.

6. Probenentnahmevorrichtung nach einem der Ansprüche 1 bis 5,
**dadurch gekennzeichnet, daß** an jedem Probeentnahme- und Mischbehälter (13, 14) eine Druckausgleichsleitung (15) für den Arbeitsdruck der Milchsammelanlage angeschlossen ist.
